Europäisches Patentamt

European Patent Office

Office européen des brevets

(11) Publication number: **0 081 492**
**B1**

(12) **EUROPEAN PATENT SPECIFICATION**

(45) Date of publication of patent specification: **16.07.86**

(21) Application number: **81902223.7**

(22) Date of filing: **10.08.81**

(86) International application number:
**PCT/US81/01063**

(87) International publication number:
**WO 82/04393 23.12.82 Gazette 82/30**

(51) Int. Cl.⁴: **A 61 K 33/04**, A 61 K 33/08

(54) METHOD AND COMPOSITION FOR TREATING ACNE.

(30) Priority: **12.06.81 US 270928**

(43) Date of publication of application:
**22.06.83 Bulletin 83/25**

(45) Publication of the grant of the patent:
**16.07.86 Bulletin 86/29**

(84) Designated Contracting States:
**FR**

(56) References cited:
**US-A-1 800 502**
**US-A-2 156 790**

**CHEMICAL ABSTRACTS, vol. 85, no. 10,
September 6, 1976, page 307, abstract no.
67502p, COLUMBUS OHIO (US)**

**Handbook of Non-Prescription Drugs, fifth
edition, pub. American Pharmaceutical
Association, 1977, pp. 317-323**

**Encyclopedia of Chemical Technology, Kirk-
Othmer, Interscience Publishers, 1964, pp. 554,
580, 581**

(73) Proprietor: **RORER INTERNATIONAL
(OVERSEAS) INC.
100 West Tenth Street
Wilmington, DE 19899 (US)**

(72) Inventor: **KLEIN, Robert Warren
1013 Union Meeting Road
Blue Bell, PA 19422 (US)**

(74) Representative: **Lecca, Jean et al
CABINET PLASSERAUD 84, rue d'Amsterdam
F-75009 Paris (FR)**

Courier Press, Leamington Spa, England.

# 0 081 492

**Description**

Field of the invention

This invention relates to a method and composition for the topical treatment of acne and oily skin. The invention also relates to a method for deodorizing compositions containing sulfurated lime solution which are intended for topical administration, and for preventing stain of skin or fabric as well as discoloration of jewelry by the composition.

Background of the invention

It is well established that acne is associated with sebum production and that androgen stimulate sebum production whereas estrogen suppress sebum production, estrogen therapy being indicated as a possible means of treating acne. Several reports indicate that oral contraceptives or the individual active estrogenic components thereof, for example, ethinyl estradiol and derivatives are useful in treating acne in both males and females. In recent years it has become apparent that estrogenic products currently in use possess certain undesirable side effects which must be set against the undoubted benefits resulting from their use. The use of estrogens for the treatment of acne in women can lead to uterine bleeding and spotting and breast tenderness. In men, estrogen administration can have a feminizing effect and may result in gynecomastia and impotence [L. F. Goodman and A. Gilman, *The Pharmacological Basis of Therapeutics*, 4th Ed., The MacMillan Company, p. 1537 (1970)]. Estrogenic therapy has been reported to give rise to other deleterious side effects. For example, diethylstilbestrol, a once widely used and well established estrogen, has been implicated as possibly being responsible for vaginal cancer and adenosis in the female offspring of pregnant women treated with the compound (*Lancet* 1975, 1960). Also ethynil estradiol and mestranol, which represent estrogenic compounds in current oral contraceptives, are now known to be involved in certain serious side effects, associated with oral contraceptives including depression [*Nature* 243, 58 (1973)], hypertension [*Am. J. Obstet. Gynecol* 112, 912, (1972)], carbohydrate and lipid abnormalities (*Lancet* 1969, p. 783), interference with blood clotting mechanism resulting in thrombosis and stroke [*Ann. Intern. Med.* 72, 111, (1970)], and jaundice [Am. J. Obstet. *Gynecol.* 119, 1965, (1974)]. Consequently, there is a need for an improved method of treating acne.

The present invention provides novel compositions for topically treating acne and oily skin with a nonsteroidal agent.

A sulfurated lime solution has been found to be effective in the treatment of various skin conditions. However, sulfurated lime solution, an amber, staining liquid yields an objectionable odor which precludes its use on the face or chest. The present invention now provides a means for deodorizing the compositions which utilize sulfurated lime solution so as to be generally acceptable for cosmetic and therapeutic use.

Fujihara et al have disclosed, in Japan Kokai 74,160,186 25 December 1975 (Chemical Abstracts, vol. 85, No. 10 of Sept. 6, 1976 p. 307, ref. No. 67502p) a process of treating waste gas containing $H_2S$, $CH_3SH$ and $Me_2S$. The clay-based adsorbent for this purpose contained a calcium hydroxide solution and 4 to 8 millimeter size attapulgite mixed into the solution. The break through time was about 10 hours. The composition, of course, was utilized for the general treatment of waste gases, and the attapulgite was not used in combination with a sulfurated lime solution.

Summary of the invention

The present invention relates to an anti-acne and anti-seborrheic pharmaceutical composition for topical administration comprising 1—20% by weight of a sulfurated lime solution and 5—50% by weight of a carrier comprising a pharmaceutically acceptable finely divided sorbent powder which contains at least one montmorillonite clay in an amount of 2 to 9% by weight based on the weight of the total composition, and at least about 1.5% by weight of attapulgite based on the weight of the total composition, said sorbent powder being present in an amount which deodorizes said composition, adsorbs skin oils, and releases an effective amount of said sulfurated lime solution to treat the patient.

Additionally, this invention relates to a method for deodorizing cosmetic and therapeutic compositions having an objectionable odoriferous sulfur-containing compound which is present in a sulfurated lime solution, which comprises incorporating in said composition an effective deodorizing amount of a pharmaceutically acceptable finely divided inorganic sorbent powder containing in combination montmorillonite clay and attapulgite, and optionally a pigment extender.

This invention further relates to a method for deodorizing a composition comprising 1—20% preferably 1—10% by weight of total composition of a sulfurated lime solution, characterized by including in said composition 5—50% by weight of a sorbent powder, said powder comprising at least 2—9 parts by weight of at least one montmorillonite clay and at least 1.5 parts by weight of attapulgite, said sorbent powder being present in an amount so as to deodorize said composition.

Still further, the composition of this invention provides the use of sulfurated lime solution without the problem of staining clothing or tarnishing jewelry.

The invention also relates to an anti-acne and anti-seborrheic pharmaceutical composition for topical administration comprising an effective amount, preferably 1—10% by weight of the composition of a sulfurated lime solution, preferably 1—10% by weight of the composition, and 5—50% by weight of a carrier comprising a pharmaceutically acceptable clay, and optionally pigment extender.

2

Detailed description of the invention

As used herein the term "acne" is intended to mean any inflammatory disease or condition of the sebaceous gland commonly occurring at puberty resulting in comedones, pustules, papules, inflamed nodules or infected cysts. A solution of sulfurated lime in combination with a finely divided sorbent powder consisting of a mixture of clays, namely, a montmorillonite clay in combination with attapulgite, has been found to be effective in reducing the amount of sebum produced by sebaceous glands.

The composition of the present invention, when administered to a patient having an acne condition or oily skin condition, represents a novel method of treating acne which offers distinct advantages over previously employed methods of treatment, for example, estrogen therapy, in that the compounds employed do not result in certain deleterious side effects resulting with estrogen therapy as will become more apparent hereinafter.

"Sulfurated lime" as used herein is commercially available and may be prepared according to the procedure disclosed in *Mellor*, Vol. III, p. 740 (1928). Generally, sulfurated lime contains not less than 55% calcium sulfides and carbonate, and the "ash" from the carbonaceous material from which it is formed.

It has been surprisingly discovered that the combination of a montmorillonite clay and attapulgite removes the odor of the sulfurated solution while releasing any perfume odor which may be present.

The clays which may be utilized in the composition of the present invention are the natural and synthetic montmorillonite clays such as the bentonites, kaolin, colloidal aluminium magnesium silicate, Fuller's earth, hectorite, smectite and saponite or mixtures thereof in combination with attapulgite.

Montmorillonite is the name of a group of clays with an expanding lattice which are members of the clay-mineral group. The montmorillonites contain aluminium silicates with some montmorillonites having some of the aluminium replaced by magnesium.

The sulfurated lime composition of the present invention can be administered in various manners to achieve the desired effect. The amount of sulfurated lime solution in the composition will vary with the severity of the acne or oily skin condition and the mode of administration. Generally the sulfurated lime solution comprises 1—20% by weight of the total composition, preferably 2—10% by weight of the total composition. The sorbent powders are employed in the amount of 5—50% by weight of composition. The sorbent powder to be an effective deodorant for the sulfurated lime solution must contain at least 2—9% by weight of a montmorillonite clay based on the weight of the total composition and at least 1.5% of attapulgite based on the weight of the total composition. The topical formulation of the present invention may contain pharmaceutically acceptable surfactants, particularly those having a detergent action for microabrasion of the uppermost layer of the skin, i.e., horny layers, to provide a smooth feeling to the skin, soothing agents such as camphor, cooling agents such as menthol, dispersing agents, penetrants, perfumes, and a conservation agent such as butylated hydroxytoluene.

The addition of calcium carbonate to the composition has been found to be advantageous for maintaining the composition's alkalinity and color. Also, it is useful in providing a micro abrasive effect so as to remove the horny layer of the skin and leave a smooth feeling.

Talc may be added to the composition to improve the slip qualities of the composition on the skin.

An effective amount of the other acne-treating agents may be incorporated in the composition such as in an amount of up to 6% by weight of the composition. These additional active ingredients include salicylic acid, resorcinol and its derivatives, retinoic acid and its derivatives, sulfur and erythromycin. Aerosol preparations containing the sulfurated lime solution and the mixture of clays together with extenders in the form of a finely grouped powder may also be employed for topical administration. The aerosols may be packaged in a pressurized aerosol container together with a gaseous or liquified propellant, for example, dichlorodifluoromethane, dichlorodifluoroethane, carbon dioxide, nitrogen, or propane with the usual adjuvant such as a suspending agent and wetting agents as may be necessary or desirable. Although the present formulation may be applied directly to the site requiring treatment, it is preferably applied in the form of a face mask so that during its application, the absorbing and adsorbing action of the sorbent powder on the face oils takes place and the sulfurated lime becomes more effective in the desired treatment of the skin condition.

The following Examples are illustrative of formulations of compositions according to this invention.

Example I

A composition for use as a face mask containing the following composition:

| | |
|---|---|
| Sulfurated lime solution (Vleminckx's solution) | 5 ml |
| Colloidal aluminium magnesium silicate (Veegum®) (a montmorillonite type clay) | 750 mg |
| Attapulgite | 250 mg |
| Sodium lauryl sulfate | 150 mg |
| Butylated hydroxytoluene | 15 mg |
| Purified water | q.s. |

was prepared as follows:

Into a beaker containing 5 ml of sulfurated lime solution, 150 mg of sodium lauryl sulfate, 15 mg of butylated hydroxytoluene and 10 ml of purified water was stirred in 750 mg of colloidal aluminium magnesium silicate and 250 mg of attapulgite until a uniform paste was formed. The composition contained essentially no sulfur odor and was suitable for forming a face mask in the treatment of acne.

Example II

A composition for the treatment of acne and seborrheic conditions having the following:

| | |
|---|---|
| Sulfurated lime solution | 20 ml |
| Hectorite clay | 1.5 g |
| Attapulgite | 0.75 g |
| Polyethylene glycol monostearate | 0.50 g |
| Purified water | q.s. |

was prepared as follows:

Into a beaker containing 20 ml of sulfurated lime solution was added 1.5 g of Laponite®, a commercially available hectorite clay and 0.75 g of attapulgite. Purified water was then heated and mixed with polyethylene glycol monostearate until uniform. Then mixed and cooled to room temperature. This was then added until a thick paste was formed that was spreadable by use of an applicator stick.

Example III

A paste for a face mask for use in the treatment of severe acne and having an anti-seborrheic effect comprising:

| | |
|---|---|
| Sulfurated lime solution | 20 ml |
| Retinoic acid | 12 mg |
| Sodium lauryl sulfate | 0.5 g |
| Bentonite | 2 g |
| Attapulgite | 1 g |
| Purified water | q.s. |

was prepared as follows:

Into a beaker containing 20 mg of sulfurate lime solution was added with stirring 0.5 g of sodium lauryl sulfate, 12 mg of retinoic acid, 1 g of attapulgite and 2 g of pulverized bentonite clay. Purified water was then added to the mixture until a paste of a desired consistency was obtained. The resulting paste may then be applied directly to the site requiring treatment. A current of warm air may then be blown on the face until the mixture dries. The composition is maintained on the face for 20 to 30 minutes and then removed by washing with water. The treatment is administered either every other day or daily until the condition treated is alleviated.

Example IV

| Ingredients | % |
|---|---|
| Vleminckx's solution | 6.00 |
| Partially acetylated polyoxyethylene lanolin ether (Laneth-10 acetate) | 2.00 |
| Decyl oleate | 0.60 |
| Alcohol | 7.00 |
| Titanium dioxide | 3.00 |
| Kaolin | 15.00 |
| Calcium carbonate | 2.30 |
| Attapulgite | 3.50 |
| Calcium silicate | 2.00 |
| Silica | 0.225 |
| EDTA | 0.10 |
| Paraben | 0.20 |
| Dioctyl sodium sulfosuccinate | 0.16 |
| Fragrance | 0.10 |
| Fatty alkanolamide | 5.00 |
| Sodium sulfate of an ethoxylated fatty alcohol | 5.00 |
| Purified water | q.s. |

The formulation was effective for the treatment of acne and oily skin conditions. Removal of the paste after drying by water washing gave a smooth feeling to the skin.

Comparison Experiment A

A sulfur-containing lotion was prepared utilizing a conventional formulation with the following ingredients:

| Ingredients | % |
| --- | --- |
| Sulfur (U.S.P.) | 5.00 |
| Cellulose gum | 0.45 |
| Propylene glycol | 3.00 |
| Attapulgite | 2.00 |
| Iron oxide | 1.20 |
| Alcohol | 20.00 |
| Fragrance | 0.10 |
| Fatty alkanolamide | 2.00 |
| Methyl paraben | 0.15 |
| Talc | 7.00 |
| Zinc oxide | 7.00 |
| Deionized water | q.s. |
| Titanium dioxide | 4.00 |

Comparison Experiment B

A lotion was prepared utilizing the formula of Experiment A except that a sulfurated lime solution (Vleminckx's solution) was utilized in lieu of sulfur.

TABLE I

| Sample | Odor | Cosmetic effect |
| --- | --- | --- |
| 1. Vleminckx's solution | Objectionable | Poor (not suitable for use) |
| 2. Elemental sulfur | Slightly pungent | Poor (could be used) |
| 3. Paste of Example IV | Pleasant | Good |
| 4. Lotion of Experiment A | Pleasant | Good |
| 5. Lotion of Experiment B | Slightly objectionable | Fair (not suitable for use) |

It was noted that elemental sulfur by itself or in a conventional carrier could be utilized by patients without any objectionable odor being present. Use of a sulfurated lime solution by itself or in a conventional carrier such as those containing elemental sulfur still produced an objectionable odor which would prevent use on the face area. A formulation prepared according to Example IV had a pleasant odor and could be utilized for skin care.

Experiment C

A. Mixtures of 6% sulfurated lime solution and the following clays were prepared with sufficient water to give paste-like consistencies:

TABLE I

| Clay | Test A (parts by wt) | Odor | Test B (parts by wt) | Odor |
|------|------|------|------|------|
| 1. Bentonite | 10 | Pungent | 20 | Pungent |
| 2. Kaolin | 10 | Pungent | 20 | Pungent |
| 3. Attapulgite | 10 | Pungent | 20 | Pungent |
| 4. Calcium silicate | 10 | Pungent | 20 | Pungent |

The pungent "rotten egg" odor of the sulfurated lime solution was evident although somewhat diminished in each of the formulations. Each mixture caused a light yellow stain when applied to fabric. Contact with silver jewelry resulted in tarnishing.

B. Mixtures were prepared according to the procedure of Part A.

TABLE II

| Clay | Test A (parts by wt) | Odor | Test B (parts by wt) | Odor |
|------|------|------|------|------|
| 1. Bentonite | 10 | Slight | 20 | Slight |
| Kaolin | 10 | Pungent | 10 | Pungent |
| 2. Bentonite | 10 | None | 20 | None |
| Attapulgite | 10 | | 10 | |
| 3. Bentonite | 10 | Slight | 20 | Slight |
| Calcium silicate | 10 | Pungent | 10 | Pungent |
| 4. Kaolin | 10 | None | 20 | None |
| Attapulgite | 10 | | 10 | |
| 5. Kaolin | 10 | Slight | 20 | Slight |
| Calcium silicate | 10 | Pungent | 10 | Pungent |
| 6. Calcium silicate | 10 | Slight | 20 | Slight |
| Attapulgite | 10 | Pungent | 10 | Pungent |

TABLE III

| Clay | Parts by wt. | Odor |
|------|------|------|
| 1. Bentonite | 9.0 | Slight |
| Attapulgite | 0.5 | Pungent |
| 2. Bentonite | 9.0 | None |
| Attapulgite | 1.5 | |
| 3. Kaolin | 9.0 | Slight |
| Attapulgite | 0.5 | Pungent |
| 4. Kaolin | 9.0 | None |
| Attapulgite | 1.5 | |

The use of 1.5 parts of attapulgite yielded a mixture which was virtually free of sulfurated lime solution odor. Mixtures (2) and (4) caused no staining of fabrics. Contact with silver, gold and chrome plated jewelry caused no tarnishing, even after 10 hours of contact.

**Claims**

1. An anti-acne and anti-seborrheic pharmaceutical composition for topical administration comprising

7

1—20% by weight of a sulfurated lime solution and 5—50% by weight of a carrier comprising a pharmaceutically acceptable finely divided sorbent powder which contains at least one montmorillonite clay in an amount of 2 to 9% by weight based on the weight of the total composition, and at least about 1.5% by weight of attapulgite based on the weight of the total composition, said sorbent powder being present in an amount which deodorizes said composition, adsorbs skin oils, and releases an effective amount of said sulfurated lime solution to treat the patient.

2. The composition according to claim 1, characterized by further including a pharmaceutically acceptable surfactant.

3. The composition according to claim 1 or 2, wherein said montmorillonite clay is selected from bentonite, hectorite, smectite, kaolin and saponite.

4. The composition according to claim 1, 2 or 3, characterized by said sorbent powder further including a compound selected from talc and calcium carbonate.

5. The composition according to any of claims 1 to 4 characterized by further including an effective amount of a compound selected from retinoic acid, salicylic acid and resorcinol.

6. An anti-acne and anti-seborrheic pharmaceutical composition for topical administration comprising an effective amount of a sulfurated lime solution and 5—50% by weight of a carrier comprising a pharmaceutically acceptable clay, and optionally a pigment extender.

7. The composition of claim 6, wherein said sulfurated lime solution comprises 1—10% by weight of said composition.

8. The composition of claim 6 or 7 characterized by further including a compound selected from salicylic acid, resorcinol, retinoic acid and derivatives thereof.

9. A method for deodorizing cosmetic and therapeutic compositions having an objectionable odoriferous sulfur-containing compound which is present in a sulfurated lime solution, which comprises incorporating in said composition an effective deodorizing amount of a pharmaceutically acceptable finely divided inorganic sorbent powder containing in combination montmorillonite clay and attapulgite.

10. The method of claim 9, wherein the composition comprises 5—50% by weight of said sorbent powder.

11. The method of claim 9 or 10, wherein said sorbent powder further includes a pigment extender.

12. The method of claim 9, 10 or 11, wherein said sorbent powder includes a clay selected from colloidal aluminum magnesium silicate, kaolin, bentonite, hectorite, smectite and Fuller's earth.

13. The method of any of claim 9 to 12, wherein said sorbent powder further includes talc and/or calcium carbonate.

14. A method for deodorizing a composition comprising 1—20% by weight of total composition of a sulfurated lime solution, characterized by including in said composition 5—50% by weight of a sorbent powder, said powder comprising at least 2—9 parts by weight of at least one montmorillonite clay and at least 1.5 parts by weight of attapulgite, said sorbent powder being present in an amount so as to deodorize said composition.

15. The method according to claim 14, characterized by said composition comprising 1—10% by weight of sulfurated lime solution and adding 5—50% by weight of finely divided powder containing at least one montmorillonite clay in an amount of 2—9 parts by weight of powder and at least 1.5 parts by weight of attapulgite, whereby said clays are present in an amount sufficient to deodorize said composition, to absorb and adsorb skin oils and to release an effective amount of sulfurated lime solution.

16. The method according to claim 14 or 15, characterized by further including a surfactant.

17. The method according to claim 14, 15 or 16, characterized by said montmorillonite clay consisting of bentonite, hectorite, smectite, kaolin and saponite.

18. The method according to any of claims 14 to 17, characterized by said sorbent powder further comprising talc and/or calcium carbonate.

## Patentansprüche

1. Pharmazeutische Anti-Akne und Anti-Seborrhöe Mischung zur örtlichen Anwendung aus 1 bis 20 Gew.-% einer sulfurierten Kalklösung und aus 5 bis 50 Gew.-% einer Trägersubstanz aus einem pharmazeutisch akzeptablen, fein zerteilten, sorbierenden Pulver, welches zumindest einen Montmorillonit-Ton in einer Menge von 2 bis 9 Gew.-% bezogen auf das Gewicht der Gesamtmischung und zumindest ungefähr 1,5 Gew.-% Attapulgit bezogen auf das Gewicht der Gesamtmischung enthält, wobei das sorbierende Pulver in einer Menge vorhanden ist, die die Mischung desodoriert, Hautfett adsorbiert und eine wirksame Menge der sulfurierten Kalklösung zur Behandlung des Patienten freisetzt.

2. Mischung nach Anspruch 1, dadurch gekennzeichnet, daß sie zusätzlich ein pharmazeutisch akzeptables oberflächenaktives Mittel (surfactant) enthält.

3. Mischung nach Anspruch 1 oder 2, worin der Montmorillonit-Ton aus Bentoniten, Hectoriten, Smectiten, Kaolinen und Saponiten ausgewählt ist.

4. Mischung nach Anspruch 1, 2 oder 3, dadurch gekennzeichnet, daß das sorbierende Pulver zusätzlich eine aus Talk und Calciumcarbonaten ausgewählte Verbindung enthält.

5. Mischung nach einem der vorangehenden Ansprüche, dadurch gekennzeichnet, daß sie zusätzlich eine wirksame Menge einer aus Retinsäuren, Salicylsäuren und Resorcinen ausgewählte Verbindung enthält.

6. Pharmazeutische Anti-Akne und Anti-Seborrhöe Mischung zur örtlichen Anwendung, die eine wirksame Menge einer sulfurierten Kalklösung und 5 bis 50 Gew.-% einer Trägersubstanz, die einen pharmazeutisch akzeptablen Ton und wahlweise einen Pigmentfüllstoff umfaßt, enthält.

7. Mischung nach Anspruch 6, worin die sulfurierte Kalklösung 1 bis 10 Gew.-% der Mischung umfaßt.

8. Mischung nach Anspruch 6 oder 7, dadurch gekennzeichnet, daß sie zusätzlich eine aus Salicylsäuren, Resorcinen, Retinsäuren und Derivaten davon ausgewählte Verbindung enthält.

9. Verfahren zur Desodorierung kosmetischer und therapeutischer Mischungen, die eine unangenehm duftende schwefelhaltige Verbindung enthalten, die in einer sulfurierten Kalklösung enthalten ist, daß das Einmünden einer wirksam desodorierenden Menge eines pharmazeutisch akzeptablen, fein zerteilten anorganischen sorbierenden Pulvers, das in Kombination Montmorillonit-Ton und Attapulgit enthält, in die Mischung umfaßt.

10. Verfahren nach Anspruch 9, worin die Mischung 5 bis 50 Gew.-% des sorbierenden Pulvers enthält.

11. Verfahren nach Anspruch 9 oder 10, worin das sorbierende Pulver zusätzlich einen Pigmentfüllstoff enthält.

12. Verfahren nach Anspruch 9, 10 oder 11, worin das sorbierende Pulver einen aus kolloidalen Aluminium Magnesium Silikaten, Kaolinen, Bentoniten, Hectoriten, Smectiten und Bleicherden ausgewählten Ton enthält.

13. Verfahren nach einem der Ansprüche 9 bis 12, worin das sorbierende Pulver Talk und/oder Calciumcarbonat enthält.

14. Verfahren zur Desodorierung einer Mischung, die 1 bis 20 Gew.-% der Gesamtmischung an sulfurierter Kalklösung enthält, dadurch gekennzeichnet, daß in der Mischung 5 bis 50 Gew.-% eines sorbierenden Pulvers beigefügt werden, wobei das Pulver zumindest 2 bis 9 Gewichtsteile zumindest eines Montmorillonit-Tones und zumindest 1,5 Gewichtsteile eines Attapulgits enthält, wobei das sorbierende Pulver in einer Menge vorhanden ist, um die Mischung zu desodorieren.

15. Verfahren nach Anspruch 14, dadurch gekennzeichnet, daß die Mischung aus 1 bis 10 Gew.-% einer sulfurierten Kalklösung und hinzugefügten 5 bis 50 Gew.-% eines fein zerteilten Pulvers, das zumindest einen Montmorillonit-Ton in einer Menge von 2 bis 9 Gewichtsteilen Pulver und zumindest 1,5 Gewichtsteile Attapulgit enthält, wobei die Tone in einer Menge vorhanden sind, die ausreicht, die Mischung zu desodorieren, Hautfette zu absorbieren und adsorbieren und eine wirksame Menge an sulfurierter Kalklösung freizusetzen.

16. Verfahren nach Anspruch 14 oder 15, gekennzeichnet durch zusätzliches Einbinden eines oberflächenaktiven Mittels (surfactant).

17. Verfahren nach Anspruch 14, 15 oder 16, dadurch gekennzeichnet, daß der Montmorillonit-Ton aus Bentoniten, Hectoriten, Smectiten, Kaolinen und Saponiten besteht.

18. Verfahren nach einem der Ansprüche 14 bis 17, dadurch gekennzeichnet, daß das sorbierende Pulver zusätzlich Talk- und/oder Calciumcarbonat enthält.

## Revendications

1. Composition pharmaceutique anti-acné et anti-séborrhéique pour l'administration topique, comprenant 1—20% en poids d'une solution de chaux sulfurée et de 5—50% en poids d'un véhicule comprenant une poudre sorbante finement divisée, pharmaceutiquement acceptable, qui contient au moins une argile de type montmorillonite dans une quantité de 2 à 9% en poids basés sur le poids de la composition totale, et au moins environ 1,5% en poids d'attapulgite basé sur le poids de la composition totale, ladite poudre sorbante étant présente dans une quantité qui désodorise ladite composition, adsorbe les matières grasses de la peau, et dégage une quantité efficace de ladite solution de chaux sulfurée pour traiter le patient.

2. Composition selon la revendication 1, caractérisée en ce qu'elle contient en plus un agent tensio-actif, pharmaceutiquement acceptable.

3. Composition selon la revendication 1 ou 2, dans laquelle ladite argile de type montmorillonite est choisie parmi la bentonite, l'hectorite, la smectite, le kaolin et la saponite.

4. Composition selon la revendication 1, 2 ou 3, caractérisée en ce que ladite poudre sorbante contient en plus un composé choisi parmi talc et le carbonate de calcium.

5. Composition selon l'une quelconque des revendications 1 à 4, caractérisée en ce qu'elle contient en plus une quantité efficace d'un composé choisi parmi l'acide rétinoïque, l'acide salicylique et le résorcinol.

6. Composition pharmaceutique anti-acné et anti-séborrhéique pour l'administration topique comprenant une quantité efficace d'une solution de chaux sulfurée et 5—50% en poids d'un véhicule comprenant une argile pharmaceutiquement acceptable et éventuellement une allonge pigmentaire.

7. Composition de la revendication 6, dans laquelle ladite solution de chaux sulfurée comprend 1—10% en poids de ladite composition.

8. Composition des revendications 6 ou 7, caractérisée en ce qu'on y incorpore en plus un composé choisi parmi l'acide salicylique, le résorcinol, l'acide rétinoïque et leurs dérivés.

9. Procédé pour désodoriser les compositions cosmétiques et thérapeutiques ayant un composé

9

contenant du souffre malodorant, qui est présente dans une solution de chaux sulfurée, lequel comprend l'étape d'addition dans ladite composition d'une quantité efficace désodorisante d'une poudre sorbante minérale finement divisée, pharmaceutiquement acceptable, contenant en combinaison de l'argile de type montmorillonite et de l'attapulgite.

10. Procédé selon la revendication 9, dans lequel la composition contient 5—50% en poids de ladite poudre sorbante.

11. Procédé selon les revendications 9 ou 10 dans lequel ladite poudre sorbante contient en plus une allonge pigmentaire.

12. Procédé selon les revendications 9, 10 ou 11, dans lequel ladite poudre sorbante contient une argile choisie parmi le silicate d'aluminium-magnésium colloïdal, le kaolin, la bentonite, l'hectorite, la smectite et la terre à foulon.

13. Procédé selon l'une quelconque des revendications 9 à 12, dans lequel ladite poudre sorbante contient en plus du talc et/ou du carbonate de calcium.

14. Procédé pour désodoriser une composition contenant 1—20% en poids de la composition totale d'une solution de chaux sulfurée, caractérisé en ce que l'on ajoute dans ladite composition 5—50% en poids d'une poudre sorbante, ladite poudre contenant au moins 2—9 parties en poids d'au moins une argile de type montmorillonite et d'au moins 1,5 partie en poids d'attapulgite, ladite poudre sorbante étant présente dans une proportion telle qu'elle désodorise ladite composition.

15. Procédé selon la revendication 14, caractérisée en ce que ladite composition comprend 1—10% en poids d'une solution de chaux sulfurée et en ce que l'on ajoute 5—50% en poids d'une poudre finement divisée contenant au moins une argile de type montmorillonite dans une proportion de 2—9 parties en poids de la poudre et d'au moins 1,5 partie en poids d'attapulgite, de manière que lesdites argiles soient présentes dans une proportion suffisante pour désodoriser ladite composition, pour absorber et adsorber les matières grasses cutanées et pour libérer une proportion efficace de solution de chaux sulfurée.

16. Procédé selon la revendication 14 ou 15, caractérisé en ce que l'on ajoute en plus un agent tensio-actif.

17. Procédé selon les revendications 14, 15 ou 16, caractérisé en ce que ladite argile de type montmorillonite est constituée par de la bentonite, de l'hectorite, de la smectite, du kaolin et de la saponite.

18. Procédé selon l'une quelconque des revendications 14 à 17, caractérisé en ce ladite poudre sorbante contient en plus du talc et/ou du carbonate de calcium.